# FASCICULE DE BREVET EUROPEEN

(11) **EP 0 521 222 B1**
(45) Date de publication et mention de la délivrance du brevet: **10.01.1996**
(21) Numéro de dépôt: 91401794.2
(22) Date de dépôt: 01.07.1991
(51) Int. Cl.: A61F 2/02

(54) **Dispositif d'implantation temporaire d'un filtre sanguin dans une veine du corps humain**
Vorrichtung zum zeitlichen Einsetzen eines Blutfilters in eine Vene des menschlichen Körpers
Device for temporary implanting a blood filter into a vein of the human body

(43) Date de publication de la demande: 07.01.1993
(73) Titulaire: Gory, Pierre, F-22000 Saint-Brieuc (FR); Bovyn, Gilles, F-22000 Saint-Brieuc (FR)
(72) Inventeur: Gory, Pierre, F-22000 Saint-Brieuc (FR); Bovyn, Gilles, F-22000 Saint-Brieuc (FR)
(74) Mandataire: Lerner, François

(56) Documents cités:
- FR-A- 2 580 504
- FR-A- 2 625 671
- FR-A- 2 643 250
- US-A- 4 643 184
- US-A- 4 873 978

## Description

La présente invention se rapporte à un dispositif d'implantation temporaire comme défini dans le preambule de la revendication 1. Un tel dispositif est connu du document FR-A-2580504.

Les filtres sanguins ont pour fonction de retenir les caillots de sang qui peuvent se former au cours de phlébites ou autres maladies cardio-vasculaires ou vasculaires, afin d'empêcher leur migration vers les artères pulmonaires où ils risqueraient de provoquer une embolie.

Les filtres généralement utilisés à cet effet ont la forme générale d'une petite ombrelle formée d'une pluralité de branches flexibles, radialement expansibles. En position de repos (état escamoté), les branches s'étendent approximativement parallèlement les unes aux autres et occupent une dimension réduite en direction radiale, ce qui autorise leur mise en place dans une veine. Une fois en place à l'intérieur de la veine, les branches se déploient automatiquement vers l'extérieur pour se bloquer contre la paroi de la veine, réalisant l'ancrage du filtre à l'endroit souhaité.

De tels filtres sont de préférence mis en place dans la veine cave inférieure, un peu en-dessous du niveau des reins.

De manière traditionnelle, l'appareillage qui permet de mettre en place le filtre comprend une tige de guidage et un mandrin qui permettent d'insérer dans la veine une gaine à la profondeur souhaitée, de telle sorte que l'extrémité de la gaine arrive à l'endroit où le filtre doit être placé. Lorsque le filtre est mis en place dans la veine cave inférieure, il est connu d'opérer l'implantation par une voie d'accès percutanée ou par "dénudation" au niveau de la veine jugulaire interne droite. La mise en place se fait donc à partir de la veine jugulaire, via la veine cave supérieure. Après mise en place de la gaine, le mandrin et la tige de guidage sont retirés ; le filtre est ensuite introduit dans la gaine au moyen d'une seringue spéciale, et est déplacé à l'intérieur de la gaine, tout au long de cette dernière, à l'aide du mandrin, celui-ci jouant alors le rôle d'un poussoir. Quand le filtre arrive à l'extrémité libre de la gaine (préalablement positionnée à l'endroit souhaité de la veine cave inférieure) il se déploie automatiquement et s'ancre dans la paroi de la veine. La gaine est ensuite enlevée et le filtre reste donc en place de manière définitive.

Le principal inconvénient de cette technique est qu'il n'est pas possible de retirer le filtre à moins de procéder à une opération chirurgicale très délicate. Malheureusement, le maintien définitif du filtre dans la veine cave est une source de complications, notamment par suite des thromboses qu'il peut déclencher ; de plus le port par le patient du filtre exige qu'il ait à vie une médicamentation anticoagulante.

Ces inconvénients sont d'autant plus regrettables que, dans de nombreuses thérapies, le maintien du filtre dans la veine n'est nécessaire que durant une période limitée, généralement de quelques semaines ou quelques mois, correspondant à une période au cours de laquelle le risque d'embolie est réel.

C'est pourquoi il a été proposé récemment une technique d'implantation temporaire du filtre, lequel peut être retiré après un certain laps de temps.

Pour cela, le mandrin qui sert à l'implantation du filtre est solidaire à demeure de ce dernier ; après implantation du filtre, le mandrin reste inséré dans la veine et ressort du corps par la voie d'accès utilisée pour l'implantation, c'est-à-dire au niveau du cou, dans la région de la veine jugulaire, pour une implantation de la veine cave. Malheureusement, le port d'une telle tige, qui est relativement rigide, est très inconfortable pour le patient ; de plus, du fait que le mandrin ressort de la peau, il constitue une source d'infections susceptibles d'entraîner des risques de complications graves, notamment des septicémies.

C'est pourquoi, la présente invention a pour but de remédier à ces inconvénients en proposant un nouveau dispositif d'implantation d'un filtre sanguin du genre évoqué plus haut, ce dispositif étant simple à manipuler et à utiliser, ceci de manière réversible, sans que le patient n'ait à courir de risques réels d'infections ou soit traumatisé par la présence du dispositif implanté au cours de toute la période au cours de laquelle il doit garder le filtre.

Dans l'ensemble de la description et des revendications, les termes "proximal(e)" et "distal(e)" sont utilisés en prenant comme référence l'endroit du corps du patient par lequel le filtre sanguin est introduit. Selon cette définition, l'extrémité proximale d'un élément est celle qui est la plus proche de l'endroit d'introduction et l'extrémité distale est celle qui en est la plus éloignée.

Le dispositif objet de l'invention est defini dans la revendication 1.

Par ailleurs, selon un certain nombre de caractéristiques avantageuses, mais non limitatives :
- les extrémités proximales dudit mandrin et de ladite gaine portent chacune une tête en forme de manchon femelle aux normes internationales LUER, la tête portée par le mandrin étant adaptée pour venir en butée contre la tête portée par la gaine, pour faciliter leur mise en place conjointe à l'intérieur de la veine ;
- le dispositif comporte un corps de seringue tubulaire apte à être emmanché sur ledit cathéter et à recevoir le filtre sanguin pour maintenir celui-ci à l'état rétracté ;
- l'extrémité proximale de la tête portée par la gaine et l'extrémité distale du corps de seringue sont pourvus de moyens d'accouplement complémentaires répondant aux normes dites "LUER-Lock" tels que filetage/taraudage facilitant l'introduction du filtre dans la gaine préalablement posée ;
- ledit organe de repérage a la forme d'un manchon destiné à être serti sur le cathéter ;
- ledit organe de repérage est un manchon dépourvu d'angles vifs, par exemple de forme ovoïde ;
- ledit organe de repérage est réalisé en matière plastique surmoulée sur une bague métallique ;
- ledit cathéter est réalisé en matière plastique, par exemple en chlorure de polyvinyle recouverte d'un matériau biocompatible tel qu'un matériau à base de silicone ;
- l'extrémité distale dudit mandrin porte un repère radio-opaque, par exemple une bague métallique ;
- l'extrémité distale du cathéter, qui porte le filtre, est fermée.

D'autres caractéristiques et avantages de l'invention apparaîtront de la description et des dessins annexés qui en présentent un mode de réalisation préférentiel.

Sur ces dessins :

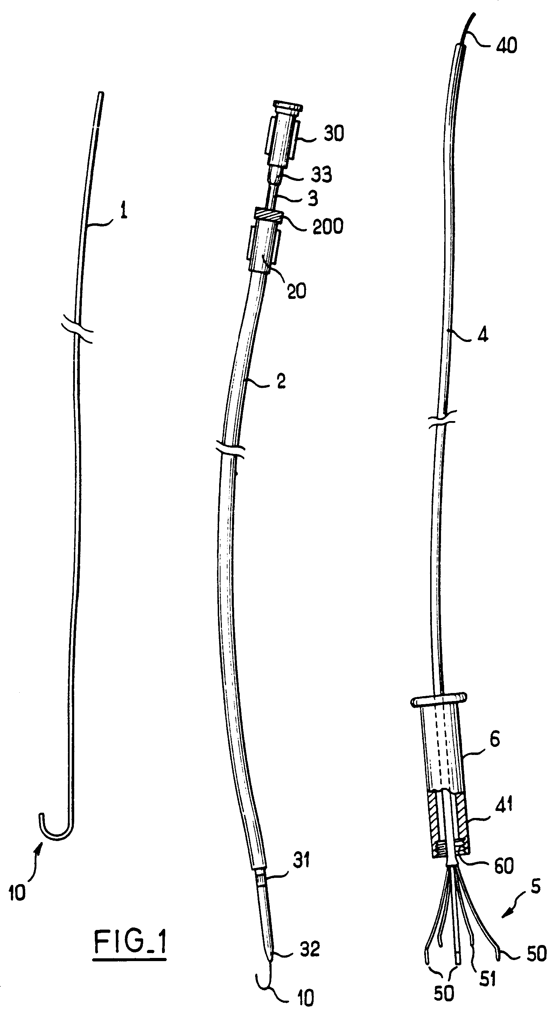

- la figure 1 représente les différents éléments constitutifs du dispositif ;
- la figure 2 représente en coupe longitudinale l'extrémité distale du cathéter équipé du filtre sanguin ;
- la figure 3 est une vue schématique illustrant la mise en place du filtre dans la veine cave inférieure d'un patient ;
- la figure 4 est une vue en coupe longitudinale de l'organe de repérage ;
- la figure 5 est une vue schématique du cathéter dont les extrémités proximales et distales sont pourvues respectivement de l'organe de repérage et du filtre.

Les différents éléments principaux constitutifs du dispositif, représentés sur les dessins, ont été référencés de la manière suivante : fil de guidage 1, gaine tubulaire 2, mandrin tubulaire 3, cathéter 4, filtre 5, câble rigidificateur 40, organe de repérage 7 et corps de seringue 6.

Le fil 1 est un fil de faible diamètre, par exemple métallique, qui est élastiquement souple, mais possède tout de même une certaine rigidité. Son extrémité distale 10 est recourbée pour former une demi-boucle, ce qui donne au fil l'allure générale de la lettre J.

La gaine 2 est un tube cylindrique à paroi mince réalisée en matière plastique, par exemple en chlorure de polyvinyle. Elle est ouverte à ses deux extrémités. A son extrémité proximale, elle porte une tête 20 en forme de manchon, également en matière plastique, dont le diamètre est plus important que le diamètre extérieur de la gaine. Cette gaine présente une certaine souplesse. Sur l'extrémité libre (côté proximal) de la tête 20, il est prévu un pas de vis 200 constitué d'un ou plusieurs filets (normes LUER-Lock).

Le mandrin tubulaire 3 consiste en une tige en matière plastique semi-rigide, dont le diamètre intérieur correspond au diamètre du fil 1, ce qui permet son emmanchement sur ce fil, tandis que son diamètre extérieur correspond au diamètre intérieur de la gaine 2, ce qui permet d'emmancher la gaine sur le mandrin.

Les emmanchements du mandrin sur le fil et de la gaine sur le mandrin sont des emmanchements prévus avec un jeu suffisant pour permettre le coulissement longitudinal relatif de ces trois éléments.

Le mandrin tubulaire 3 porte à son extrémité proximale une tête 30 en forme de manchon aux normes LUER ; cette dernière présente un prolongement 33, cylindrique ou légèrement conique, adapté pour venir s'emboîter dans un logement complémentaire prévu dans la tête de gaine 20 ; après emboîtement, la gaine 2 et le mandrin 3 sont donc parfaitement solidaires l'un de l'autre si bien qu'en manipulant l'une des deux têtes 20 ou 30 seulement, il est possible de déplacer l'ensemble mandrin et gaine. L'extrémité distale 32 du mandrin présente une forme conique à sommet arrondi, cette forme douce évitant les agressions au cours de l'implantation du dispositif.

A faible distance de l'extrémité 32, il est prévu sur le mandrin 3 un repère radio-opaque 31, par exemple constitué par une petite bague métallique. Les longueurs relatives de la gaine et du mandrin sont déterminées pour que, après enfoncement complet du mandrin dans la gaine (tête 30 en butée contre tête 20) le repère 31 ressorte juste à l'extrémité distale de la gaine.

Le cathéter 4 est constitué par un tube très souple (non rigide) dont le diamètre extérieur est sensiblement plus petit que celui de la gaine 2.

Le cathéter 4 est réalisé dans une matière plastique recouverte d'un matériau bio-compatible tel qu'un matériau à base de silicone ; la matière plastique est par exemple du chlorure de polyvinyle. Avantageusement, le mandrin est rendu radio-opaque, par exemple par inclusion de particules de sulfate de baryum dans le matériau qui le constitue.

Comme déjà dit, le cathéter 4 a une forme tubulaire, et il peut recevoir dans sa lumière centrale un câble rigidificateur 40 de diamètre correspondant, ce câble étant par exemple constitué par un fil très mince en acier ressort enroulé sur lui-même hélicoïdalement sur toute sa longueur.

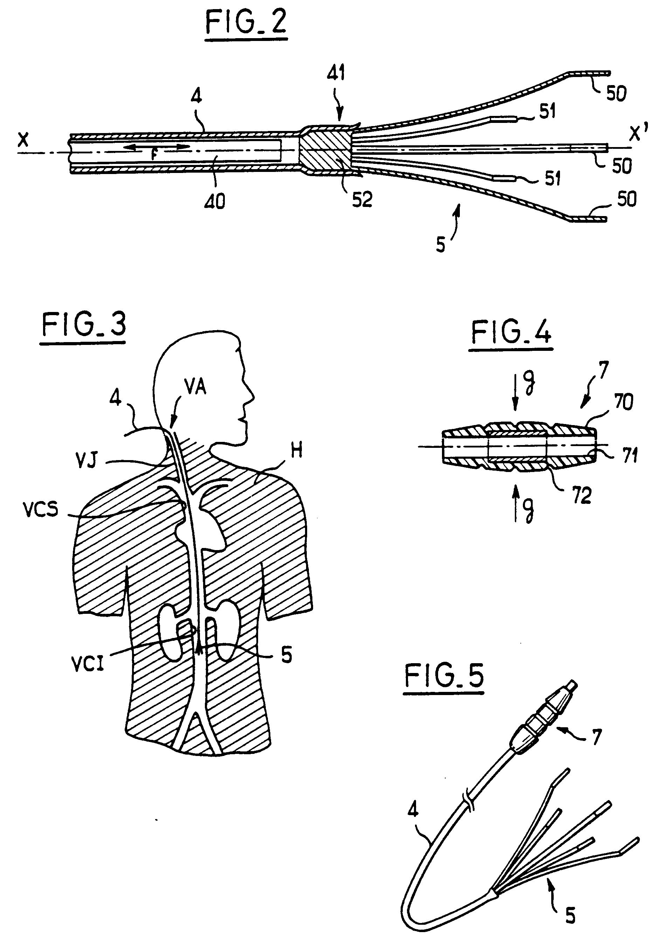

Comme on le voit plus particulièrement à la figure 2, l'extrémité distale du cathéter 4 est fermée par un élément 52 qui forme partie du filtre 5. Le coulissement du câble rigidificateur 40 dans le cathéter 4 (coulissement figuré par la double flèche f à la figure 2) est donc limité en direction distale, de sorte que le câble sert de poussoir lors de l'introduction du cathéter, comme cela sera expliqué plus loin.

Le filtre 5 est un filtre de type connu en soi, du genre décrit dans le préambule de la présente description. Dans le mode de réalisation représenté, le filtre a la forme d'une armature d'ombrelle qui comporte huit branches constituées par de fines lamelles métalliques flexibles. Il est prévu quatre branches longues 50 alternées avec quatre branches plus courtes 51, selon une répartition angulaire régulière à 45°.

Ces branches sont encastrées à leur extrémité proximale dans le bouchon de fermeture 52 du cathéter 4. Leur extrémité distale est légèrement recourbée de manière à présenter une direction sensiblement parallèle à l'axe longitudinale XX' du filtre et du cathéter. On comprend ainsi, lorsque le filtre se trouve à l'état déployé (état représenté sur les figures) les extrémités libres des branches 50 et 51 viennent se plaquer correctement contre la paroi de la veine sans risque d'agresser celle-ci.

Le dispositif comporte également un corps de seringue en matière plastique 6, lequel a la forme d'un manchon tubulaire pouvant coulisser sur le cathéter 4. La longueur du corps 6 est un peu plus grande que la longueur des branches du filtre 5. Son diamètre intérieur correspond sensiblement au diamètre intérieur de la tête de gaine 20 et de la gaine 2.

L'extrémité distale du corps 6 est taraudée de manière à présenter un filet 60 complémentaire du pas de vis 200 de la tête de gaine 20 selon les normes LUER.

L'organe de repérage 7, représenté aux figures 4 et 5 comprend un corps 70 en forme de petit manchon réalisé en matière plastique souple. Ce manchon, dépourvu d'angles vifs, a la forme générale d'une petite olive dont la partie centrale plus renflée est surmoulée sur une bague métallique 72.

Comme cela est figuré à la figure 4 par les flèches g, le sertissage de l'organe de repèrage 7 sur une tige préalablement emmanchée dans le trou central 71 du corps 70, et par conséquent la fixation de l'organe 7 sur cette tige, peut être facilement obtenu par écrasement de la zone centrale du manchon et déformation de la bague 72. Comme on le verra plus loin, il est ainsi possible de fixer de manière simple l'organe 7 à l'extrémité proximale du cathéter 4.

Nous allons maintenant expliquer de quelle manière le dispositif qui vient d'être décrit est utilisé pour la mise en place d'un filtre sanguin dans la veine cave inférieure d'un corps humain, ceci de manière réversible, le filtre pouvant être aisément enlevé ultérieurement.

L'implantation du filtre se fait sous anesthésie locale du patient, lequel est référencé H à la figure 3. De manière classique, l'opérateur commence par ménager dans le cou une voie d'accès percutanée VA de la veine jugulaire interne droite VJ, ou par dénudation.

Dans une première phase, il introduit dans la veine jugulaire le fil de guidage 1. Sous observation radiologique, rendue possible par la radio-opacité du fil, il fait descendre ce dernier dans la veine jugulaire puis lui fait emprunter la veine cave supérieure VCS et ensuite la veine cave inférieure VCI. Du fait que l'extrémité distale de la tige de guidage est recourbée, cette extrémité ne constitue pas une cause d'accrochage ni de traumatisme au cours de son déplacement. Cette opération cesse lorsque l'extrémité 10 est arrivée un peu au-delà de la zone ou le filtre doit être implanté, en-dessous des bifurcations d'irrigation rénale.

Il réalise alors une petite incision d'élargissement de part et d'autre du point d'entrée du guide pour faciliter l'opération suivante.

Dans une seconde phase, la tête 30 étant en butée contre la tête 20, l'opérateur emmanche sur l'extrémité proximale du fil 1 (qui ressort bien entendu de la veine jugulaire) l'ensemble unitaire formé du mandrin 3 et de la gaine 2 ; il fait descendre doucement cet ensemble le long du fil 1, jusqu'à ce que le repère radio-opaque 31 atteigne la zone prévue pour l'ancrage du filtre.

Dans une troisième phase, il retire de la gaine 2 le fil 1 et le mandrin 3.

Dans une quatrième phase, alors que le corps de seringue 6 coiffe le filtre 5, lequel se trouve par conséquent à l'état replié, il accouple ce corps de seringue sur la tête 20 de la gaine à l'aide de moyens de vissage complémentaires 60, 200.

Dans une cinquième phase, le câble rigidificateur 40 se trouvant à l'intérieur du cathéter 4, il fait descendre cet ensemble de sorte que le filtre 5, toujours à l'état rétracté, est transféré tout d'abord dans la tête 20 puis dans la gaine 2. L'effort de poussée qui est appliqué par l'opérateur à l'extrémité distale de l'ensemble cathéter 4 - câble 40 est transmis correctement au filtre en raison du rôle rigidificateur du câble, si bien que le filtre descend progressivement le long de la gaine 2. Ceci serait difficile en l'absence du câble 40 en raison de la souplesse du cathéter (qui est semi-rigide).

Lorsque le filtre est arrivé à l'extrémité distale de la gaine 2, il se déploie automatiquement par suite de la flexibilité des branches 50, 51, lesquelles viennent s'appliquer contre les parois de la veine cave à l'endroit souhaité, ce qui réalise l'ancrage du filtre.

Dans une sixième phase, l'opérateur retire la gaine 2 de la veine.

Dans une septième phase, il retire du cathéter 4 le câble 40, puis sectionne le cathéter à l'extérieur de la veine jugulaire, à faible distance de celle-ci. Ce sectionnement peut être effectué commodément en raison du caractère facilement sécable du cathéter, par exemple au moyen d'une paire de ciseaux chirurgicaux ordinaires.

Dans une huitième phase, l'opérateur emmanche sur l'extrémité proximale dépassante du cathéter l'organe de repérage 7, puis le fixe par sertissage à l'aide d'un outil approprié, par exemple une pince.

Enfin l'opérateur réalise par la petite incision d'élargissement une logette sous le muscle peaucier du cou, où il enfouit l'organe 7. Puis il referme la voie d'accès VA de manière classique, de telle sorte que l'organe 7 reste emprisonné sous la peau après suture. Il n'y a donc pas de risque d'infection par voie transcutanée au niveau du cou.

Il faut noter de plus que du fait que le cathéter 4 est fermé à son extrémité distale, tout risque de reflux de sang dans le cathéter en direction de la voie d'accès est évité ; en outre, l'écrasement de l'organe de repèrage 7 sur le cathéter provoque également l'obturation de ce dernier à son extrémité proximale.

En raison de sa souplesse, le cathéter ne gêne aucunement le patient, ni ne perturbe ses activités ; il est complètement "oublié" par le patient durant toute la phase où le filtre doit être conservé en place dans la veine cave inférieure. La durée de conservation du cathéter et du filtre peut être portée de quelques semaines à plusieurs mois grâce à l'invention, alors qu'elle n'est que de deux à trois semaines dans les techniques de pose temporaire mises en oeuvre jusqu'ici, avec les risques d'infection liés à cette technique.

A tout moment, il est possible de contrôler la position de l'organe 7, soit en palpant la peau du patient, soit sous radiographie. Lorsque le filtre doit être enlevé, il suffit d'ouvrir à nouveau la voie d'accès VA, et de faire sortir le filtre de la veine en tirant sur l'extrémité du cathéter 4. La forme particulière du filtre, qui peut se contracter librement vers l'intérieur, autorise son déplacement le long des veines VCI, VCS et VJ.

On donnera ci-après, à titre purement indicatif les principales dimensions possibles du dispositif.

Le mandrin 3 et la gaine 2 pourront avoir une longueur de l'ordre 50 à 65 cm, le cathéter 4 une longueur de l'ordre de 60 à 80 cm, le fil 1 et le câble 40 des longueurs de l'ordre de 80 à 100 cm.

Le fil de guidage 1 et le câble rigidificateur 40 pourront avoir un diamètre de l'ordre de 0,5 à 0,7 mm ; le mandrin 3 et la gaine 2 pourront avoir respectivement des diamètres extérieurs de l'ordre de 3,5 et de 4,2 mm, tandis que le cathéter 4 pourra avoir un diamètre extérieur de l'ordre de 2 mm. Les branches longues 50 du filtre 5 pourront avoir une longueur de l'ordre de 40 mm, tandis que ses branches courtes 51 pourront avoir une longueur de l'ordre de 25 mm.

Après pose du dispositif, la longueur effective du cathéter, c'est-à-dire la longueur comprise entre le filtre 5 et l'organe de repèrage 7 sera généralement compris entre 40 et 55 cm.

L'ensemble des éléments constitutifs du dispositif objet de l'invention sont destinés à être conditionnés dans un même emballage stérile, à usage unique, en vue de leur commercialisation et de leur livraison aux centres de soins médico-chirurgicaux.

## Revendications

1. Dispositif d'implantation temporaire, dans une veine du corps humain, et en particulier dans la veine cave inférieure (VCI) d'un filtre sanguin (5) du type élastiquement expansible en direction radiale, composé d'une part
d'un fil de guidage élastique et de faible diamètre (1) destiné à être inséré dans la veine sensiblement jusqu'à la position ou le filtre doit être implanté par une voie d'accès percutanée (VA) ou par dénudation, d'une gaine tubulaire (2) à paroi mince et d'un cathéter (4) en forme de tube souple facilement sécable, dont l'extrémité distale porte à demeure le filtre sanguin (5), caractérisé en ce que ledit dispositif est composé d'autre part d'un mandrin tubulaire semi-rigide (3) apte à être emmanché sur le fil de guidage (1) et déplacé le long de celui-ci sensiblement jusqu'à la position ou le filtre doit être implanté,
ladite gaine tubulaire (2) à paroi mince pouvant être emmanchée sur le mandrin (3) et mise en place à l'intérieur de la veine (VCI) sensiblement jusqu'à la position ou le filtre doit être implanté par suite du déplacement du mandrin (3) le long du fil de guidage (1),
d'un câble rigidificateur (40) pouvant être inséré de façon amovible dans le cathéter (4), ce qui permet de déplacer à l'intérieur de ladite gaine (2) préalablement posée le cathéter (4) et son filtre (5) à l'état rétracté, jusqu'à ce que ce dernier ressorte à l'extrémité distale de la gaine et se déploie alors automatiquement pour s'ancrer contre la paroi de la veine (VCI) et
d'un organe de repérage (7) destiné à être fixé à l'extrémité proximale du cathéter (4) après enlèvement dudit câble rigidificateur et sectionnement du cathéter au niveau de la voie d'accès (VA) et à s'enfouir sous le peau du corps humain après suture.

2. Dispositif selon la revendication 1, caractérisé en ce que les extrémités proximales dudit mandrin (3) et de ladite gaine (2) portent chacune une tête (30, 20) en forme de manchon, la tête (30) portée par le mandrin (3) étant adaptée pour venir en butée contre la tête (20) portée par la gaine, pour faciliter leur mise en place conjointe à l'intérieur de la veine.

3. Dispositif selon l'une des revendications 1 ou 2, caractérisé en ce qu'il comporte un corps de seringue tubulaire (6) apte à être emmanché sur ledit cathéter (4) et à recevoir le filtre sanguin (5) pour maintenir celui-ci à l'état rétracté.

4. Dispositif selon les revendications 2 et 3 prises en combinaison, caractérisé en ce que l'extrémité proximale de la tête (20) portée par la gaine (2) et l'extrémité distale du corps de seringue (6) sont pourvues de moyens d'accouplement complémentaires tels que filetage (200)/taraudage (60) facilitant l'introduction du filtre (5) dans la gaine (2) préalablement posée.

5. Dispositif selon l'une des revendications 1 à 4, caractérisé en ce que ledit organe de repérage (7) a la forme d'un manchon destiné à être serti sur le cathéter (4).

6. Dispositif selon l'une des revendications 1 à 5, caractérisé en ce que ledit organe de repérage (7) est un manchon dépourvu d'angles vifs, par exemple de forme ovoïde.

7. Dispositif selon l'une des revendications 1 à 6, caractérisé en ce que ledit organe de repérage est réalisé en matière plastique surmoulée sur une bague métallique.

8. Dispositif selon l'une des revendications 1 à 7, caractérisé en ce que ledit cathéter (4) est réalisé en matière plastique, par exemple en chlorure de polyvinyle recouverte d'un matériau biocompatible tel qu'un matériau à base de silicone.

9. Dispositif selon l'une des revendications 1 à 8, caractérisé en ce que l'extrémité distale dudit mandrin (3) porte un repère radio-opaque, par exemple une bague métallique (31).

10. Dispositif selon l'une des revendications 1 à 9, caractérisé en ce que l'extrémité distale (41) du cathéter (4), qui porte le filtre (5), est fermée.

## Claims

1. Device for temporarily implanting, in a vein of the human body, and in particular in the inferior vena cava (IVC), a blood filter (5) of the type which is elastically expandable in the radial direction, composed, on the one hand, of an elastic guide wire of small diameter (1) intended to be inserted into the vein substantially as far as the position where the filter is to be implanted via a percutaneous access route (AR) or by denudation, of a thin-walled tubular sheath (2) and of a catheter (4) in the form of an easily divisible flexible tube whose distal end permanently bears the blood filter (5), characterised in that the said device is composed, on the other hand, of a semi-rigid tubular mandrel (3) capable of being fitted onto the guide wire (1) and displaced along the latter, substantially as far as the position where the filter is to be implanted, the said thin-walled tubular sheath (2) being capable of being fitted onto the mandrel (3) and of being positioned inside the vein (IVC) substantially as far as the position where the filter is to be implanted by means of displacement of the mandrel (3) along the guide wire (1), of a strengthening cable (40) capable of being inserted in a removable manner in the catheter (4), this making it possible to displace, inside the said previously positioned sheath (2), the catheter (4) and its filter (5) in the retracted state, until the latter emerges at the distal end of the sheath and then spreads open automatically and anchors against the wall of the vein (IVC) and of a locating member (7) intended to be fixed at the proximal end of the catheter (4) after removal of the said strengthening cable and cutting of the catheter at the level of the access route (AR) and to be buried under the skin of the human body after suturing.

2. Device according to Claim 1, characterised in that the proximal ends of the said mandrel (3) and of the said sheath (2) each bear a head (30, 20) in the form of a sleeve, the head (30) borne by the mandrel (3) being adapted to come into abutment against the head (20) borne by the sheath, in order to facilitate their joint positioning inside the vein.

3. Device according to one of Claims 1 and 2, characterised in that it comprises a tubular syringe body (6) capable of being fitted onto the said catheter (4) and of receiving the blood filter (5) in order to hold the latter in the retracted state.

4. Device according to Claims 2 and 3 taken together, characterised in that the proximal end of the head (20) borne by the sheath (2) and the distal end of the syringe body (6) are provided with complementary coupling means, such as a threading (200)/tapping (60), facilitating the introduction of the filter (5) into the previously positioned sheath (2).

5. Device according to one of Claims 1 to 4, characterised in that the said locating member (7) has the form of a sleeve intended to be crimped onto the catheter (4).

6. Device according to one of Claims 1 to 5, characterised in that the said locating member (7) is a sleeve having no sharp corners, for example of ovoid shape.

7. Device according to one of Claims 1 to 6, characterised in that the said locating member is made of plastic material cast on a metal ring.

8. Device according to one of Claims 1 to 7, characterised in that the said catheter (4) is made of plastic material, for example polyvinyl chloride, covered with a biocompatible material such as a silicone-based material.

9. Device according to one of Claims 1 to 8, characterised in that the distal end of the said mandrel (3) bears a radiopaque marker, for example a metal ring (31).

10. Device according to one of Claims 1 to 9, characterised in that the distal end (41) of the catheter (4), which bears the filter (5), is closed.

## Patentansprüche

1. Vorrichtung zum temporären Implantieren eines Blutfilters (5) von in radialer Richtung elastisch ausdehnbarem Typ in eine Ader des menschlichen Körpers und insbesondere in die untere Hohlvene (VCI), mit einerseits
einem Führungsfaden, der elastisch ist, einen geringen Durchmesser (1) hat und der dazu bestimmt ist, in die Ader im wesentlichen bis zu der Position, wo der Filter implantiert werden soll, durch einen durchstoßenen Zugangsweg (VA) oder durch Entblößung eingeführt zu werden, einer rohrförmigen Hülse (2) mit dünner Wand und einem Katheter (4) in Form eines weichen, leicht teilbaren Rohres, dessen entferntes Ende den Blutfilter (5) bleibend trägt, **dadurch gekennzeichnet,** daß
die Vorrichtung andererseits mit einem rohrförmigen, halbstarren Dorn (3) gebildet ist, der geeignet ist, auf den Führungsfaden (1) gesteckt und entlang diesem, im wesentlichen bis zu der Position verschoben zu werden, wo der Filter implantiert werden soll, wobei die rohrförmige Hülse (2) mit dünner Wand auf den Dorn (3) gesteckt und im Inneren der Vene (VCI) im wesentlichen bis zu der Position, wo der Filter implantiert werden soll, infolge der Verschiebung des Dorns (3) entlang des Führungsfadens (1), an den Platz gebracht werden kann,
mit einem Versteifungskabel (40) gebildet ist, das in abnehmbarer Weise in den Katheter (4) eingefügt werden kann, was erlaubt, im Inneren der vorher angeordneten Hülse (2) den Katheter (4) und seinen Filter (5) in eingezogenem Zustand zu verschieben, bis letzterer an dem entfernten Ende der Hülse hinaustritt und sich dann automatisch ausbreitet, um sich gegen die Wand der Vene (VCI) zu verankern und
mit einem Markierungsorgan (7) gebildet ist, das dazu bestimmt ist, an dem nahen Ende des Katheters (4) nach Beseitigung des Versteifungskabels und Abtrennen des Katheters an dem Zugangsweg (VA) befestigt zu werden und sich unter der Haut des menschlichen Körpers nach dem Zusammennähen zu verbergen.

2. Vorrichtung nach Anspruch 1, **dadurch gekennzeichnet,** daß die nahen Enden des Dorns (3) und der Hülse (2) jedes einen Kopf (30, 20) in der Form einer Muffe tragen, wobei der von dem Dorn (3) getragene Kopf (30) geeignet ist, gegen den von der Hülse getragenen Kopf (20) in Anschlag zu kommen, um ihr gemeinsames Anbringen im Inneren der Vene zu erleichtern.

3. Vorrichtung nach einem der Ansprüche 1 oder 2, **dadurch gekennzeichnet,** daß sie einen rohrförmigen Spritzenkörper (6) aufweist, der geeignet ist, auf dem Katheter (4) aufgesetzt zu werden und den Blutfilter (5) aufzunehmen, um diesen in zurückgezogenem Zustand zu halten.

4. Vorrichtung nach den Ansprüchen 2 und 3 in Kombination, **dadurch gekennzeichnet,** daß das nahe Ende des von der Hülse (2) getragenen Kopfes (20) und das entfernte Ende des Spritzenkörpers (6) mit zusätzlichen Kupplungsmitteln, wie zum Beispiel Außengewinde (200)/Innengewinde (60), versehen sind, welche das Einführen des Filters (5) in die vorher angeordnete Hülse (2) erleichtern.

5. Vorrichtung nach einem der Ansprüche 1 bis 4, **dadurch gekennzeichnet,** daß das Markierungsorgan (7) in der Form einer Muffe dazu bestimmt ist, auf dem Katheter (4) aufgesetzt zu werden.

6. Vorrichtung nach einem der Ansprüche 1 bis 5, **dadurch gekennzeichnet,** daß das Markierungsorgan (7) eine Muffe ohne scharfe Winkel, zum Beispiel oval ist.

7. Vorrichtung nach einem der Ansprüche 1 bis 6, **dadurch gekennzeichnet,** daß das Markierungsorgan aus einem auf einem Metallring abgeformten Kunststoffmaterial hergestellt ist.

8. Vorrichtung nach einem der Ansprüche 1 bis 7, **dadurch gekennzeichnet,** daß der Katheter (4) aus einem Kunststoffmaterial hergestellt ist, zum Beispiel aus Polyvinylchlorid, das mit einem biokompatiblen Material, wie zum Beispiel einem Material auf Silikonbasis, bedeckt ist.

9. Vorrichtung nach einem der Ansprüche 1 bis 8, **dadurch gekennzeichnet,** daß das entfernte Ende des Dorns (3) eine strahlenundurchlässige Markierung wie zum Beispiel einen metallischen Ring (31) trägt.

10. Vorrichtung nach einem der Ansprüche 1 bis 9, **dadurch gekennzeichnet,** daß das entfernte Ende (41) des Katheters (4), welches den Filter (5) trägt, geschlossen ist.
